# EUROPEAN PATENT APPLICATION

(11) **EP 1 082 951 A2**
(43) Date of publication of application: **14.03.2001**
(21) Application number: 00500191.2
(22) Date of filing: 24.08.2000
(51) Int. Cl.: A61F 5/02

(54) **A spinal load relief device**

(30) Priority: 08.09.1999 ES 9902006
(71) Applicant: Munoz Saiz, Manuel, E-28017 Madrid (ES)
(72) Inventor: Munoz Saiz, Manuel, E-28017 Madrid (ES)

(57) **Abstract**

A spinal load relief device that consists of the use of a rigid or semi-rigid, flexible, compact or hollow girdle or corset of slightly truncated cone oval shape, in two parts, one formed by two walls or portions, one in front (18) and the other behind (19), usually thin and elastic, extending from the waist to the armpit, and the other formed by two walls or portions (10 and 11) which are thicker, and which bear the effort the armpit and the pelvis or waist, curving slightly to adapt to the chest and running from thickened rounded edges (13 and 14) at the top, adapted to the armpit, to the lower edges (8 and 9), which are also thickened, with recess (15 and 16) inside the lower side walls which surround, cover, adapt to and rest on the outside upper sides of the pelvic ilium crest (4 and 5), fitting anatomically on to them to bear the effort or weight of the upper half of the body between the armpit and the pelvis or waist, rather than on the spine.

## Description

### FIELD OF THE INVENTION.

This invention refers to a girdle, waistcoat or corset type device for use in spinal rehabilitation or therapy.

### PRIOR ART.

There are corsets, girdles, etc. and devices to protect the abdomen, waist and spine which help to counteract body efforts, stresses or inclinations but which only very slightly reduce the weight of the head, chest and upper limbs on the vertebrae.

With the spine vertical, or when standing or sitting, the weight of the head, chest and upper limbs- i.e. the upper half of the body- is borne by the spine and so by each of the vertebrae and the discs between them, with only a small portion being borne by the muscles. At the same time, the spine is supported on the sacrum or central pelvis which in turn sits on the two ilium bones on each femur, tibia and foot.

The present invention overcomes those disadventages.

### DECRIPTION OF THE INVENTION.

This inventions consists of the use of a rigid or semi-rigid, flexible, compact or hollow girdle, waistcoat or corset of slightly truncated cone oval shape, in two parts, one formed by two walls or portions, one in front and the other behind, usually thin and elastic, extending from the waist to the armpit, and the other formed by two walls or portions which are thicker, and which bear the effort the armpit and the pelvis or waist, curving slightly to adapt to the chest and running from thickened rounded edges at the top, adapted to the armpit, to the lower edges, which are also thickened, with recess inside the lower side walls which surround, cover, adapt to and rest on the outside upper sides of the pelvic ilium crest, fitting anatomically on to them to bear the effort or weight of the upper half of the body between the armpit and the pelvis or waist, rather than on the spine.

Apart from reducing the weight on the spine, stretching is possible both when vertical and when horizontal or lying down.

An elastic tubular girdle can be used around and to support the side elements or walls which are, in this case, independent.

Instead of the front and side walls, elastic securing strips, also linking said side elements, may be used.

The side elements may have curved recess at the top to adapt to the armpits.

In a variant of the bottom side elements of the device, these may be more rounded, in this case inserting and resting in the hollow of the waist, like a belt.

The bottom side elements of the device may also rest in the hollows between the trochanter major and the ilium bones, like a belt.

The side elements may each take the form of two inter-connected zones or portions which may be extended or adjusted together by means of studs, screws or rods.

The front element may be substituted by two small curved fins, which may be flexible, and which adapt to the sides.

The front portion may take the form of a narrow elastic band.

The rigid front and rear elements may have rotating fins in the front area.

In one variant, the side elements do not reach the armpits and adapt to the chest cavity pressing on the sides.

The device may take the form of a waistcoat in the upper part.

The side elements may be covered with a layer of soft material such as foam rubber, felt, etc. where they rest on the armpits, ilium crest and waist.

The side elements or walls may be laterally flexible and may be rigid or semi-rigid, and they may be compact or hollow and inflatable.

If the front elements are not elastic, the device must open at the front, and may closed by clasps, belts, velcro strips, tying, cords, buttons, elastic band or similar systems.

The side elements must not press laterally and must merely adapt and hold, to allow breathing without difficulty.

Waist, chest, armpit support and height or conicity may vary in size. In all cases, it adapts to the individual shape of each user and so must be available in a number of sizes.

The device may also have flexible internal ribs.

The elements are made of thermoplastic fabrics and materials, foam rubber of various strengths, with the addition of screws, rods or strips made of metal or thermo-plastic material.

Adventages for persons with spine lesions, those under major strains, who carry weights, who must stand for stand long periods, or who need to strech their spine while seated, for growth and also for healthy individuals and can, as well, be worn while you lie in bed.

### BRIEF DESCRIPTION OF THE DRAWINGS

Figure 1 shows a front, schematic, partial, cross-section view of a human body and its skeleton.

Figure 2 shows a front, schematic, partial, cross-section view of a human body and its skeleton with the device of the invention.

Figure 3, 6, 8, 9, 10, 11 and 12 show front, schematic, partial, cross-section views of a human body and its skeleton, variants of the device of the invention.

Figure 4 and 7 show side views of the device of the invention.

Figure 5 shows a top view of the device of the invention.

Figure 13 shows a top view of a variant of the device of the invention.

### DETAILED DESCRIPTION OF THE PREFERRED EMBODIMENTS

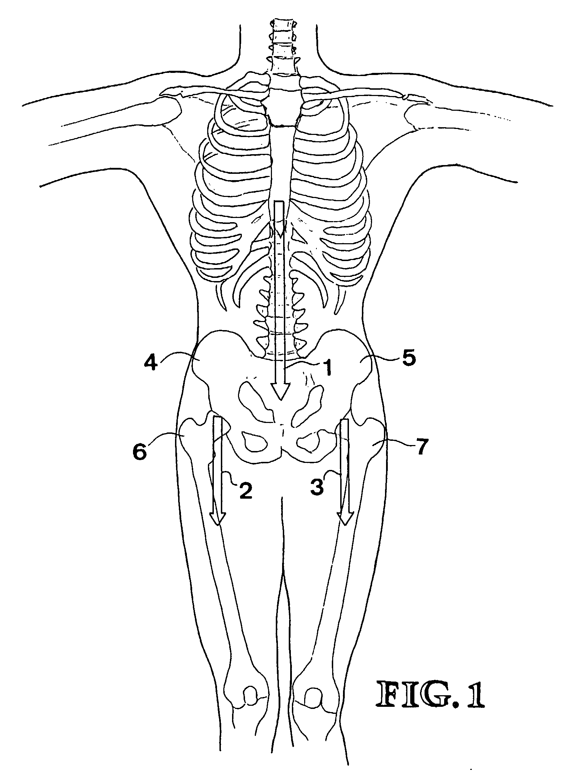

Figure 1 consists of the force or weight of the upper half of the body 1, that is borne by the spine and so by each of the vertebrae and the discs between them, said spine is supported on the sacrum or. central pelvis which in turn sits on the two ilium bones 4 and 5, forces or weights 2 and 3, on both femurs 6 and 7, tibias and feet.

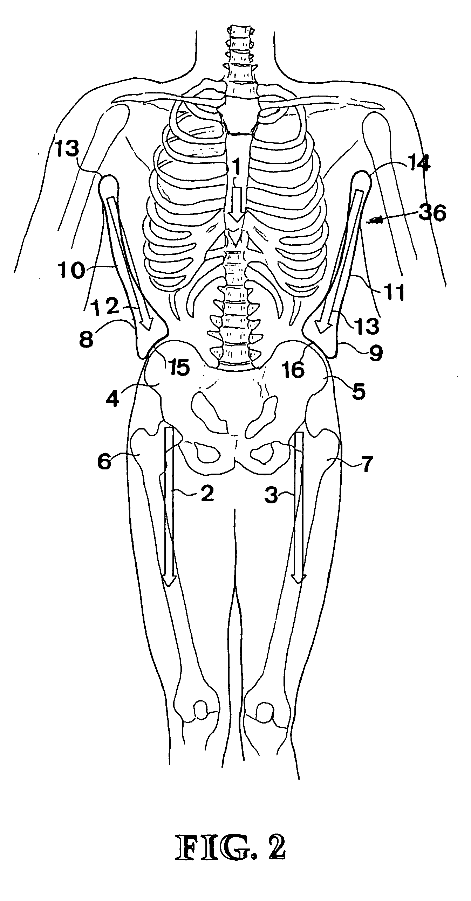

Figure 2 consists of the force or weight 1 that bear the spine, the hollow device or girdle 36, formed by two walls or portions 10 and 11, which are thicker, and which bear the efforts or weights 12 and 13 between the armpit and the pelvis or waist, curving slightly to adapt to the chest and running from thickened rounded edges 13 and 14 at the top, adapted to the armpit, to the lower edges 8 and 9, which are also thickened, with recess 15 and 16 inside the lower side walls which surround, cover, adapt to and rest on the outside upper sides of the pelvic ilium crest 4 and 5, fitting anatomically on to them to bear the effort or weight of the upper half of the body between the armpit and the pelvis or waist, rather than on the spine. At the same time, the spine is supported on the sacrum or central pelvis which in turn sits on the two ilium bones 4 and 5, forces or weights 2 and 3, on both femurs 6 and 7, tibias and feet.

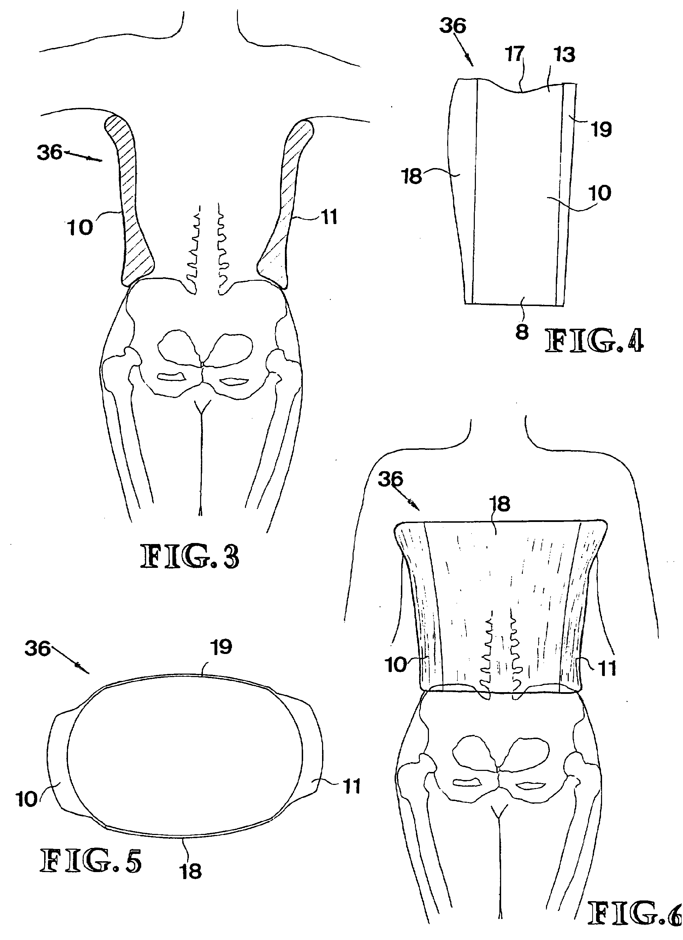

Figure 3 consists of the compact device 36 formed by two side walls or portions 10 and 11.

Figure 4 consists of the device 36, the lower thickened rounded edge 13 at the top of the side wall or portion 10, the side elements may have curved recess 17 at the top to adapt to the armpits, and the front 18 and rear 19 elastic walls.

Figure 5 consists of the device 36, the side wall or portions 10 and 11, and the front 18 and rear 19 elastic walls.

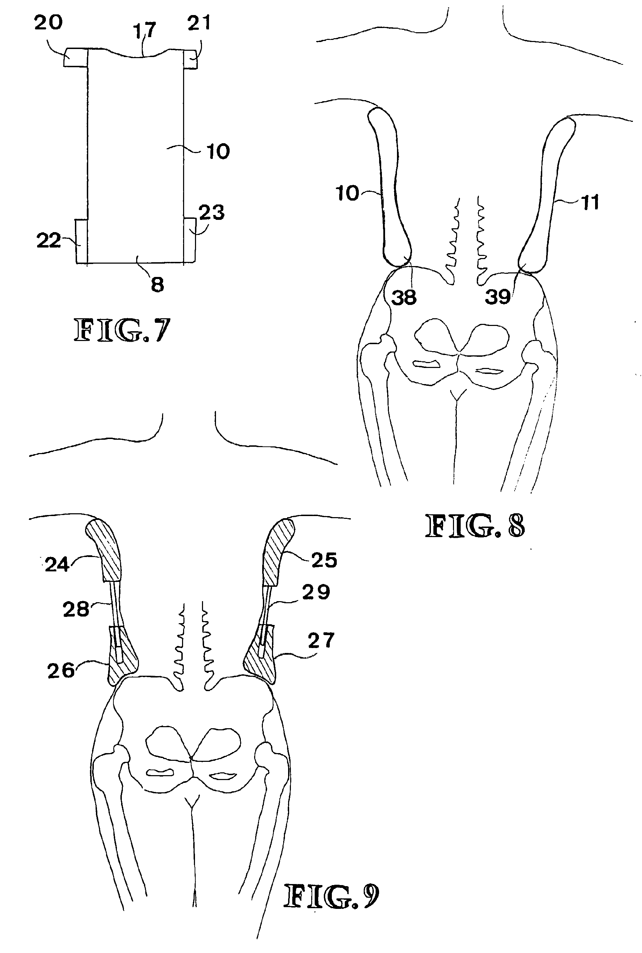

Figure 6 consists of the device 36, the side walls or portions 10 and 11 and the front elastic wall 18.

Figure 7, consists of the thickened lower edge 8, the side elements with curve recess 17 at the top, to adapt to the armpit, the front 20 and 22 and rear 21 and 23 elastic securing strips, and the side element 10.

Figure 8 consists of the bottom side elements 38 and 39, they are more rounded, in this case inserting and resting in the hollow of the waist, like a belt.

Figure 9 consists of the side elements where each take the form of two inter-connected zones or portions 24 and 26, and 25 and 27, which may be extended or adjusted together by means of studs, screws or rods (28 and 29).

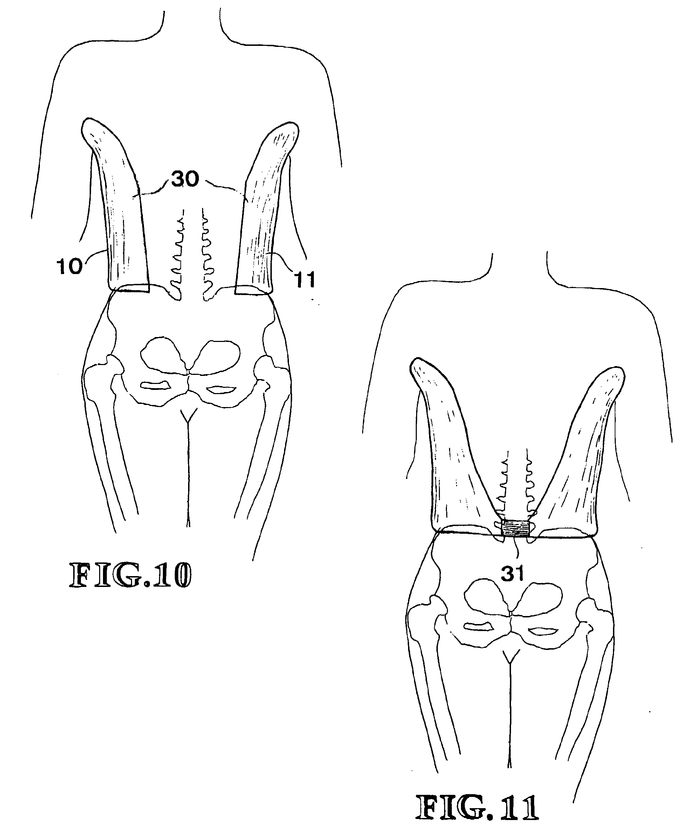

Figure 10 consists of the side elements 10 and 11, and the front element with two small curved fins 30, which may be flexible, and which adapt to the user sides.

Figure 11 conists of the narrow elastic band 31, that is placed in the front zone.

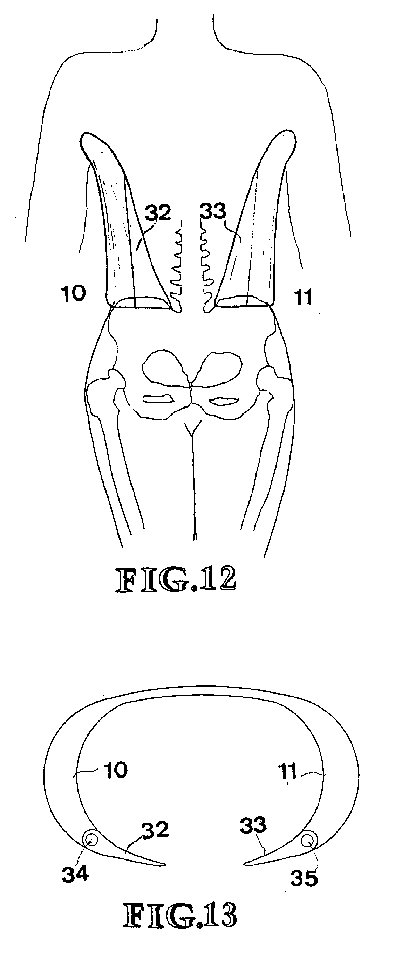

Figure 12 and 13 consists of the side elements 10 and 11 and the front and rear elements, which have in the front area, rotating fins 32 and 33 around shafts 34 and 35.

## Claims

1. A spinal load relief device that consists of the use of a rigid or semi-rigid, flexible, compact or hollow girdle or corset of slightly truncated cone oval shape, in two parts, one formed by two walls or portions, one in front (18) and the other behind (19), usually thin and elastic, extending from the waist to the armpit, and the other formed by two walls or portions (10 and 11) which are thicker, and which bear the effort the armpit and the pelvis or waist, curving slightly to adapt to the chest and running from thickened rounded edges (13 and 14) at the top, adapted to the armpit, to the lower edges (8 and 9), which are also thickened, with recess (15 and 16) inside the lower side walls which surround, cover, adapt to and rest on the outside upper sides of the pelvic ilium crest (4 and 5), fitting anatomically on to them to bear the effort or weight of the upper half of the body between the armpit and the pelvis or waist, rather than on the spine.

2. A spinal load relief device according to claim 1, wherein it is used an elastic tubular girdle around and to support the side elements or walls which are, in this case, independent.

3. A spinal load relief device according to claim 1, wherein are used elastic securing strips (20, 21, 22 and 23), also linking said side elements.

4. A spinal load relief device according to claim 1, wherein the side elements have curved recess (17) at the top to adapt to the armpits and the side elements are laterally flexible.

5. A spinal load relief device according to claim 1, wherein the bottom side elements of the device (38 and 39), these are more rounded, in this case inserting and resting in the hollow of the waist, like a belt, and the bottom side elements of the device rest in the hollows between the trochanter major and the ilium bones, like a belt.

6. A spinal load relief device according to claim 1, wherein each of the side elements take the form of two inter-connected zones or portions which are extended or adjusted together (24 with 26, and 25 with 27) by means of studs, screws or rods (28 and 29).

7. A spinal load relief device according to claim 1, wherein the front element is substituted by two small curved fins (30), that are flexible, and which adapt to the sides.

8. A spinal load relief device according to claim 1, wherein the front portion takes the form of a narrow elastic band (31).

9. A spinal load relief device according to claim 1, wherein the rigid front element has rotating fins.

10. A spinal load relief device according to claim 1, wherein the side elements are covered with a layer of soft material such as foam rubber, felt, etc. where they rest on the armpits, ilium crest and waist.

11. A spinal load relief device according to claim 1, wherein the front elements are opened and closed by clasps, belts, velcro strips, tying, cords, buttons, elastic band or similar systems.

12. A spinal load relief device according to claim 1, wherein are used hollow and inflatable side, front and rear elements.
